# EUROPEAN PATENT SPECIFICATION

(11) **EP 1 494 030 B1**
(45) Date of publication and mention of the grant of the patent: **27.11.2013**
(21) Application number: 04015270.4
(22) Date of filing: 29.06.2004
(51) Int. Cl.: G01N 33/543, G01N 33/569

(54) **Sample pretreatment solution for influenza virus test by immunochromatography**
Lösung zur Vorbehandlung eines Grippevirentests basierend auf Immunchromatographie
Solution pour le prétraitement d'un test pour un virus de grippe basé sur l'immunochromatographie

(30) Priority: 30.06.2003 JP 2003188065; 30.06.2003 JP 2003188066
(43) Date of publication of application: 05.01.2005
(73) Proprietor: SYSMEX CORPORATION, Kobe-shi, Hyogo 651-0073 (JP)
(72) Inventor: Imoarai, Takeshi, Kobe-shi Hyogo 651-2116 (JP); Furutani, Motoi, Kobe-shi Hyogo 651-2116 (JP); Aki, Masako, Kobe-shi Hyogo 654-0152 (JP)
(74) Representative: HOFFMANN EITLE

(56) References cited:
- EP-A1- 1 248 106
- EP-A1- 1 306 671
- WO-A-01/44815
- WO-A-03/053477
- US-A- 5 208 074

## Description

### BACKGROUND OF THE INVENTION

### Field of the Invention

The present invention relates to a sample pretreatment solution for immunochromatographic tests. In particular, the present invention relates to a sample pretreatment solution for testing a sample for influenza virus.

### Discussion of the Related Art

The influenza virus is an RNA virus with a diameter of 80-120 nm and has an envelope which is covered with projections of two types of enzyme proteins, i.e. HA (hemagglutinin) and NA (neuraminidase). HA is a hemagglutinating antigen, which binds to cell surface sialic acid upon attachment to and entry into human cells and plays an important role in incorporation of the virus particles into cells. NA shows the activity to cleave sialic acid when the virus particle detaches from the cell surface in the late stage of the infection, and it serves to acquire infectivity. The antigenicity is determined by the combination of HA and NA, and is roughly categorized into three types, A, B and C. Furthermore, type A viruses are known to have subtypes, such as Hong Kong-type. In type A, a new subtype emerges every decade or more and causes an antigenic shift pandemic. The antigenicity slightly changes every year even in the same subtype. Thus, it is necessary in the influenza virus tests to find out a common site that is detectable irrespective of antigenic variations in influenza viruses.

To provide a precise method for diagnosing and treating influenza infection, it is desirable to obtain the test result in a rapid way, since patients with influenza infection often recover in a few days. Currently, tissue culture methods for detection of antigens and assay methods, including hemagglutination inhibition (HI), complement fixation (CF), indirect fluorescence antibody methods (IFA) and the like as an antibody test, are in practical use.

There have been a number of reports of methods for easily utilizing antigen-antibody reactions so far. For example, an assay using immunochromatography is known, in which an obtained sample is merely impregnated into a testing device containing an antibody of interest to determine the presence or absence or the amount of the antigen (USP4861711 and USP5602040). In this method, a porous membrane such as nitrocellulose sheet is used, in which a specific antibody against a particular antigen of interest is absorbed at one end of the membrane and another specific antibody that also binds exclusively to the particular antigen is immobilized in the middle part of the porous membrane in a zonal manner. The specific antibody impregnated at one end is colored in advance. When a sample solution is applied to the end portion of the porous membrane into which the specific antibody is impregnated, an antigen reacting with the specific antibody contained in the sample solution, if any, binds to the specific antibody and migrates all together by capillary action, with the colored particles attached, through the porous membrane toward the other end, which is opposite to the sample application site. During the migration, when the sample solution passes through the zonal area in which another specific antibody is mobilized, the antigen is captured by this specific antibody on the porous membrane and then pigmented zones appear in the porous membrane, which reveals the presence and amount of the antigen of interest.

Application of this technique may enable rapid test for influenza and is useful. Influenza tests are usually carried out using nasal discharge, sputum or throat swab as samples. The samples for which the aforementioned immunochromatographic technique is available are required to be able to pass through a porous membrane by capillary action in principle. However, nasal discharge and throat swab cannot be subjected to the test due to occlusion of the pores of the porous membrane. Nasal discharge and throat swab contain a highly viscous substance mucin, which fills the pores of porous membrane as well as mediates aggregation of epithelial adherent cells exfoliated from living organs.

For example, the prior art already discloses that a kit for pretreatment of saliva which comprises aqueous solution containing sodium hydroxide, tris (hydroxymethyl) aminomethane buffer containing tartaric acid and/or citric acid, andnonionic surfactant and/or amphoteric surfactant, in which kit the surfactants are premixed in the aqueous solution and/or the buffer, or separated from the aqueous solution and the buffer is used for identification or quantification of mutans streptococcus present in human saliva samples by immunochromatography (Publication of Unexamined Application: JP-A 2002-357599). However, no disclosure has been given with regard to influenza testing.

On the other hand, WO02/10744 discloses, as a method for pretreatment of samples for influenza testing, treatment with a sample pretreatment solution containing a surfactant, at least one substance selected from reducing agents, and organic acid or a salt thereof.

### SUMMARY

Use of a first sample pretreatment solution for influenza virus tests by immunochromatography, which comprises a nonionic surfactant and at least 0.3 M alkali metal ion according to claim 12.

A first method for detecting influenza virus using immunochromatography, which comprises the steps of preparing samples; and treating the samples with the sample pretreatment solution comprising a nonionic surfactant and at least 0.3 M alkali metal ion according to claim 1.

A first influenza virus test kit comprising the sample pretreatment solution comprising a nonionic surfactant and at least 0.3 M alkali metal ion; and an immunochromatographic device according to claim 10.

The use of a second sample pretreatment solution for testing samples for influenza virus by immunochromatography, which comprises a thiocyanate compound according to claim 13.

A second method for detecting influenza virus using immunochromatography, which comprises the steps of preparing samples; and treating the samples with the sample pretreatment solution comprising a thiocyanate compound according to claim 5.

A second influenza virus test kit comprising the sample pretreatment solution compsising a thiocyanate compound and an immunochromatographic device according to claim 11.

### BRIEF DESCRIPTION OF THE DRAWINGS

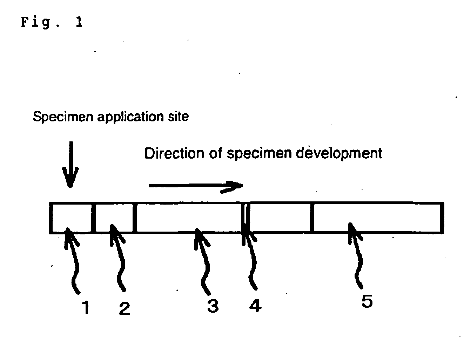

FIG. 1 shows a schematic diagram of a strip for immunochromatography.

### DETAILED DESCRIPTION

There is no particular limitation on the influenza viruses to be tested. Any viruses generally defined as influenza viruses may be tested. Specifically, any of the types A, B and C may be included. Mutant viruses or new mutant viruses that will emerge in the future maybe included as long as they are classified as influenza viruses.

### (Samples)

There is no particular limitation on the samples to be tested, as long as they are obtainable from living organisms and can be mixed with influenza virus. Preferably, the samples are, for example, nasal discharge, sputum and/or throat swab. There is no particular limitation on the methods for collecting thesesamples. Any known method may be employed. Specifically, a cotton swab may be used to take a sample of nasal discharge, sputum and/or throat swab.

### (Sample Pretreatment Solution)

There is no particular limitation on the nonionic surfactant contained in the first sample pretreatment solution. Preferably, a polyoxyethylene surfactant, and more preferably an ether surfactant may be used. More specifically, a single surfactant or a mixture of two or more surfactants may be preferably used which are selected from the group consisting of polyoxyethylene alkyl phenyl ethers, such as polyoxyethylene (9) octylphenyl ether, polyoxyethylene (10) octylphenyl ether, and polyoxyethylene (9) nonylphenyl ether; polyoxyethylene sorbitan fatty acid esters, such as polyoxyethylene sorbitan monolaurate and polyoxyethylene sorbitan monooleate; copolymers of polyoxyethylene/ polyoxypropylene; and polyoxyethylene alkyl ethers.

The nonionic surfactant may be contained in the sample pretreatment solution at a concentration of 0.05-2 (v/v) %, preferably 0.1-0.5 (v/v) %, and more preferably approximately 0.3 (v/v) %.

Alkali metal ions that may be contained in the first sample pretreatment solution include, but are not limited to, lithium⁺ (Li⁺), sodium⁺ (Na⁺), potassium⁺ (K⁺), rubidium⁺ (Rb⁺), cesium⁺ (Cs⁺) and francium⁺ (Fr⁺). Preferably, sodium and potassium may be used. The alkali metal ions may be used alone or in a combination of two or more ionic species. There is no particular limitation on the compound that may give rise to these alkali metal ions. For example, a single compound or a mixture of two or more compounds may be used which are selected from the group consisting of sodium chloride, potassium chloride, sodium hydroxide, potassium hydroxide, EDTA sodium salt and sodium azide. The alkali metal ion is required to be contained in the sample pretreatment solution at a concentration of at least 0.3 M, preferably 0.4 M, and more preferably 0.45 M or higher. However, the sensitivity of the immunochromatography may be adversely affected when 2 M or higher concentration of alkali metal ion is added. Thus, preferably, 1.5 M or lower, more preferably 1.0 M or lower concentration is adopted.

There is no particular limitation on the thiocyanate compound contained in the second sample pretreatment solution. In addition to thiocyanic acid (HNCS), aqueous solution of thiocyanate ester or thiocyanate may be used. The constituent salts of the thiocyanic acids include inorganic bases containing metals such as sodium and potassium; and organic bases such as ammonium salt. The constituent salts further include hydrates and solvates of these salts. Specifically, sodium thiocyanate, potassium thiocyanate, ammonium thiocyanate, guanidine thiocyanate and the like are included. Preferably, potassium thiocyanate or guanidine thiocyanate is used.

The thiocyanate compound may be contained in the sample pretreatment solution at a concentration of at least 0.1 M, preferably 0.125-1.0 M, and more preferably 0.125-0.375 M.

In addition to the components described above, other components such as buffer for maintaining an optimal pH of 5-9 for the reaction and organic acids may be contained in the first sample pretreatment solution.

In addition to the components described above, other components such as surfactants, buffer formaintaining anoptimal pH of 5-9 for the reaction and organic acids may be contained in the second sample pretreatment solution.

A conventional method may be used for treating the samples. For example, 0.1-0.2 ml of a sample may be added to 0.5-1.0 ml of the sample pretreatment solution and mixed well by shaking. Likewise, a cotton swab with which a sample such as nasal discharge was removed is soaked into the sample pretreatment solution and mixed well with the solution. The samples treatedwith the sample pretreatment solution are expediently referred to as "specimens" herein.

The specimens obtained using the sample pretreatment solution and the treatment method may be subjected to tests such as identification and quantification of influenza by antigen-antibody reaction using conventional immunochromatography.

### (Immunochromatography)

The principle of immunochromatography is schematically illustrated in Fig. 1, although this technique is already well-known.

In Fig. 1, the colored particles labeled with an anti-influenza virus antibody are retained in the labeling component (2) andananti-influenzavirusantibodyisimmobilized in the influenza virus detection site (4).

A sample treated as described above and prepared as a specimen is dropped on the specimen application component (1), and the specimen is developed toward the absorption component (5) through the chromatography membrane support (3). When the influenza virus of interest is contained in the specimen, the influenza virus is reacted with the colored latex particles labeled with anti-influenza virus antibody to form a complex, which is captured in the influenza virus detection site (4), in which an anti-influenza virus antibody is immobilized. The captured complex is observed as a pigmented band. The amount of the influenza virus contained in the specimen can be estimated by the color tone or such of the band emerged in the site (4).

Any antibodies may be applied to the labeling component (2) and the influenza virus detection site (4) as long as they recognize different site of the influenza virus. These antibodies may be obtained using any conventional method. For example, the method for establishing hybridomas by cell fusion according to Kohler and Milstein (Kohler G and C. Milstein, Continuous cultures of fused cells secreting antibody of predefined specificity, Nature, 256: 495-497, 1975) may be used. The antibodies may be obtained by mere immunization of an animal using an antigen, followed by purification of its serum. Any particles known to be available for immunochromatography may be used as the labeled colored particles in the labeling component (2). For example, gold colloid and colored latex particles may be used. The chromatography membrane support (3) may be a membrane typically used for immunochromatography, generally a porous membrane, and specifically a nitrocellulose membrane may be used.

### (Kit)

The sample pretreatment solution described above and an immunochromatographic device for detection of influenza virus may be combined to provide a test kit. Specifically, such a test kit comprises the sample pretreatment solution, an immunochromatographic device and optionally cotton swabs for sampling.

### Examples

The present invention is specifically illustrated below with reference to Examples, but it is not to be construed as being limited thereto.

### Example 1

### Effect of Nonionic Surfactant (NP40)

This example was made for the purpose of verifying the effect of different concentrations of a nonionic surfactant (NP40) in the sample pretreatment solution on the background on the chromatography membrane support.

### 1) Composition of the sample pretreatment solution

The sample pretreatment solution was prepared by adding Nonidet P-40 (NP40: the product name of polyoxyethylene (9) octylphenyl ether) at a concentration of 0, 0.05, 0.1, 0.2, 0.4 or 0.8 (v/v) % to a solution containing 100 mM citric acid, 0.4 M NaCl and 10 mM dithiothreitol (pH 6.0).

### 2) Samples and treatment thereof

To prepare a specimen, a nasal discharge-filled cotton swab was soaked in a container containing about 0.8 ml of the sample pretreatment solution and mixed with the sample pretreatment solution. Subsequently, the specimen solution was allowed to stand and then filtered through a membrane filter (pore diameter= 1 µm). Thus, specimens for the test were prepared. The nasal discharge samples were subjected to the MDCK cell culture (J. Clin. Microb. 28(6): 1308-1313 (1990)) and those determined as positive for influenza viruses A and B were used for the subsequent experiment.

### 3) Immunochromatography

Regarding the materials used for the immunochromatographic strip (Fig. 1), glass fiber filter was used for the specimen application component (1), polyvinyl-treated glass fiber filter retaining blue stained latex particles sensitized with a commercially available anti-influenza virus antibody was used for labeling component (2), a nitrocellulose membrane was used for the chromatography membrane support (3) , and a composite filter of glass fiber and cellulose was used for the absorption component (5). The detection site (4) was sensitized with a commercially available anti-influenza virus antibody. The samples confirmed to be influenza-positive in the culture assay were dropped on the immunochromatographic device pretreated using a conventional method and subjected to chromatography.

The immunochromatography was performed for 20 minutes, after 0.2 ml of the each test specimen pretreated with the sample pretreatment solution containing each concentration of NP40 was put on the immunochromatographic device.

The results are shown in Table 1. Since the chromatography membrane support was stained blue of the latex particles and the background was incompatible in the absence of NP-40, it could not be determined whether the specimens were positive for influenza A or B by immunochromatography. By contrast, when the specimens were pretreated with the sample pretreatment solution containing 0.05 (v/v) % or higher concentration of NP40, the chromatography membrane support was not stained and had a favorable background. Thus, determination of influenza A or B was easy.

**Table 1**

| **Concentration of NP40 (%)** | **Background** | **Specimens positive for influenza virus A or B** | |
|---|---|---|---|
| | | **A** | **B** |
| **0%** | **Incompatible** | **Indeterminable** | |
| **0.05%** | **Compatible** | 1+ | 1+ |
| **0.10%** | **Compatible** | 1+ | 1+ |
| **0.20%** | **Compatible** | 1+ | 1+ |
| **0.40%** | **Compatible** | 1+ | 1+ |
| **0.80%** | **Compatible** | 1+ | 1+ |

### Example 2

### Effect of Various Nonionic Surfactants (Background)

This example was made for the purpose of verifying the effect of different nonionic surfactants contained in the sample pretreatment solution on the background on the chromatography membrane support.

The sample pretreatment solution was prepared by adding each nonionic surfactant shown in Table 2 at a concentration of 0.1 (v/v) % to a solution containing 100 mM citric acid, 0.4 M NaCl and 10 mM dithiothreitol (pH 6.0).

The samples and treatment thereof, and immunochromatography were performed as described in Example 1.

As a result, coloration of the background was reduced when each nonionic surfactant shown in Table 2 was used, and accurate determination was made.

**Table 2**

| Results from Determination of Background | | | | |
|---|---|---|---|---|
| Product Name | Structure Name | | Concentration | Determination |
| NP40 | Polyoxyethylene (9) octylphenyl ether | Nonionic | 0.1% | Compatible |
| Triton X-100 | Polyoxyethylene (10) octylphenyl ether | Nonionic | 0.1% | Compatible |
| Tween 20 | Polyoxyethylene (20) sorbitan monolaurate | Nonionic | 0.1% | Compatible |
| HS-210 | Polyoxyethylene (10) octylphenyl ether | Nonionic | 0.1% | Compatible |
| Nonion A-10R | Polyoxyethylene/ polyoxypropylene copolymer | Nonionic | 0.1% | Compatible |
| Emulgen 909 | Polyoxyethylene (9) nonylphenyl ether | Nonionic | 0.1% | Compatible |
| Brij 97 | Polyoxyethylene (10) oleyl ether | Nonionic | 0.1% | Compatible |
| No surfactant | | | | Incompatible |
| Compatible: Coloration of the chromatography membrane support was not observed and blue colored lines were visible at the determination time. | | | | |

### Example 3

### Effect of Various Nonionic Surfactants (Influenza Test)

Like Example 2, this example was made for the purpose of verifying the effect of different nonionic surfactants contained in the sample pretreatment solution on the chromatography.

The sample pretreatment solution, the samples and treatment thereof, and the immunochromatography were performed as described in Example 2.

As a result, the specimens were determined as positive in all the surfactants, when the nonionic surfactants shown in Table 3 were used.

**Table 3**

| Results from Determination of Influenza viruses | | | | |
|---|---|---|---|---|
| Product Name | Structure Name | | Concentration | Determination |
| NP40 | Polyoxyethylene (9) octylphenyl ether | Nonionic | 0.1% | ++ |
| Triton X-100 | Polyoxyethylene (10) octylphenyl ether | Nonionic | 0.1% | ++ |
| Tween 20 | Polyoxyethylene (20) sorbitan monolaurate | Nonionic | 0.1% | + |
| HS-210 | Polyoxyethylene (10) octylphenyl ether | Nonionic | 0.1% | ++ |
| Nonion A-10R | Polyoxyethylene/ polyoxypropylene copolymer | Nonionic | 0.1% | ++ |
| Emulgen 909 | Polyoxyethylene (9) nonylphenyl ether | Nonionic | 0.1% | ++ |
| Brij 97 | Polyoxyethylene (10) oleyl ether | Nonionic | 0.1% | ++ |
| No surfactant | | | | - |

### Example 4

### Effect of addition of various concentrations of NaCl

### (Determination of Influenza A and B)

This example was made for the purpose of verifying the effect of different concentrations of sodium chloride (NaCl) in the sample pretreatment solution on the chromatography.

### 1) Composition of the sample pretreatment solution

The sample pretreatment solution was prepared by adding NaCl at the concentrations indicated in Table 4 to a solution containing 0.1 (v/v) % NP40, 100 mM citric acid, 10 mM dithiothreitol, and 10 mM EDTA (pH 6.0).

### 2) Samples and treatment thereof

Three samples of nasal discharge determined as negative for influenza A and B by culture assay, as well as cultured types A and B viruses as positive controls were pretreated according to the method described in Example 1.

### 3) Immunochromatography

Immunochromatography was performed as described in Example 1.

The results are shown in Tables 4 and 5. The negative specimens were determined as positive in the absence of NaCl, and addition of NaCl was verified to inhibit the nonspecific reaction. The positive controls were determined as positive in the presence of NaCl.

**Table 4**

| Determination of Influenza Virus Type A | | | | |
|---|---|---|---|---|
| **NaCl** | Samples negative for influenza virus | | | Cultured type-A virus |
| **(M)** | **001** | **002** | **003** | |
| **0** | **+** | **+** | **+** | **+** |
| **0.2** | **±** | **±** | **±** | **+** |
| **0.4** | **-** | **-** | **±** | **+** |
| **0.6** | **-** | **-** | **±** | **+** |
| **0.8** | **-** | **-** | **-** | **+** |
| **1.0** | **-** | **-** | **-** | **+** |

**Table 5**

| Determination of Influenza Virus Type B | | | | |
|---|---|---|---|---|
| **NaCl (M)** | Samples negative for influenza virus | | | Cultured type-B virus |
| | **001** | **002** | **003** | |
| **0** | + | + | + | + |
| **0.2** | ± | ± | ± | + |
| **0.4** | - | - | ± | + |
| **0.6** | - | - | ± | + |
| **0.8** | - | - | - | + |
| **1.0** | - | - | - | + |

### Example 5

### Effect of addition of various compounds to the sample pretreatment solution

### 1) Composition of the sample pretreatment solution

The sample pretreatment solution was prepared by adding the compounds indicated in Table 6 at the concentration of 0.5 M to a solution containing 0.1 (v/v) % NP40 (polyoxyethylene (9) octylphenyl ether), 100 mM citric acid and 0.5 M NaCl (pH 6.0).

### 2) Samples and treatment thereof

Three samples of nasal discharge determined as negative for influenza A and B by culture assay, physiological saline as negative control, cultured types A and B viruses as positive controls were pretreated according to the method described in Example 1.

### 3) Immunochromatography

Immunochromatography was performed as described in Example 1.

**Table 6**

| Nonspecific Reaction Inhibitory Effect of Treatment with the Sample Pretreatment Solution Containing Different Compounds. | | | | | | |
|---|---|---|---|---|---|---|
| | Negative control | Positive control | | Samples from patients negative for influenza viruses | | |
| | | Influenza A | Influenza B | 1 | 2 | 3 |
| No additive | - | + | + | + | + | + |
| Choline chloride | - | + | + | + | + | + |
| Guanidine thiochyanate | - | + | + | - | - | - |
| Potassium thiochyanate | - | + | + | - | - | - |
| Guanidine hydrochloride | - | + | + | + | + | + |

| | | | | | | |
|---|---|---|---|---|---|---|
| *The concentration of each additive was 0.5 M. | | | | | | |

The results show that the pretreatment with the sample pretreatment solution containing guanidine thiochyanate or potassium thiochyanate, among the compounds shown in Table 6, gave negative results for all the samples determined as negative by culture assay. This verifies that treatment of samples with the sample pretreatment solution containing a thiocyanate compound inhibits the nonspecific reaction. The positive controls were determined as positive even if thiocyanate compounds were added.

### Example 6

### Effect of Potassium Thiocyanate

This example was made for the purpose of verifying the effect of different concentrations of potassium thiocyanate (KSCN) contained in the sample pretreatment solution on the background on the chromatography membrane support. 1) Composition of the sample pretreatment solution

The sample pretreatment solution was prepared by adding KSCN at a concentration of 0, 0.100, 0.125, 0.250 or 0.375 M to a solution containing 0.1 (v/v) % NP-40 (polyoxyethylene (9) octylphenyl ether), 100 mM citric acid and 0.15 M NaCl (pH 6.0).

The samples and treatment thereof, and immunochromatography were performed as described in Example 5.

**Table 7**

| Effect of potassium thiocyanate (KSCN) | | | | | | | | |
|---|---|---|---|---|---|---|---|---|
| KSCN | Negative control | Positive control | | Samples from patients negative for influenza viruses | | | | |
| (M) | | Influenza A | Influenza B | ① | ② | ③ | ⑤ | ⑥ |
| 0 | - | ++ | ++ | + | + | + | + | ± |
| 0.100 | - | ++ | ++ | ± | + | + | ± | - |
| 0.125 | - | ++ | ++ | - | ± | ± | - | - |
| 0.250 | - | ++ | ++ | - | - | - | - | - |
| 0.375 | - | + | + | - | - | - | - | - |

As a result, as shown in Table 7, nonspecific reaction was observed in the absence of KSCN, and the nonspecific reaction was prevented by addition of KSCN. The positive controls were determined as positive even if KSCN was added.

## Claims

1. A method for detecting influenza virus, which comprises the steps of providing a sample pretreatment solution comprising a nonionic surfactant and at least 0.3 M but less than 1.0 M alkali metal ion; preparing an assay sample by mixing the said sample pretreatment solution and a sample selected from the group consisting of nasal discharge, sputum and throat swab; and detecting influenza virus in the assay sample by using immunochromatography.

2. The method of claim 1, wherein said nonionic surfactant is a polyoxyethylene surfactant.

3. The method of claim 2, wherein said polyoxyethylene surfactant is selected from the group consisting of polyoxyethylene alkyl phenyl ethers, polyoxyethylene alkyl ethers, polyoxyethylene sorbitan fatty acid esters and polyoxyethylene/ polyoxypropylene copolymers.

4. The method according to any of claims 1-3, wherein a concentration of said nonionic surfactant ranges from 0.05 to 2 (v/v) %.

5. The method according to any of claims 1-4, which comprises a thiocyanate compound.

6. The method of claim 5, wherein said thiocyanate compound is contained at a concentration of at least 0.1 M.

7. The method of claim 5 or 6, wherein said thiocyanate compound is selected from the group consisting of thiocyanic acid, thiocyanate esters and thiocyanates.

8. The method according to any of claims 5-7, wherein said thiocyanate compound is selected from the group consisting of sodium thiocyanate, potassium thiocyanate, ammonium thiocyanate and guanidine thiocyanate.

9. The method according to any of claims 1-8, wherein said assay sample preparing step comprises filtering the mixture through a membrane filter with a pore diameter of 1 µm.

10. An influenza virus test kit comprising a sample pre-treatment solution comprising a nonionic surfactant and at least 0.3 M but less than 1.0 M alkali metal ion and an immunochromatographic device for detecting influenza virus.

11. The test kit of claim 10, wherein the sample pre-treatment solution comprises a thiocyanate compound.

12. Use of a sample pretreatment solution for preparing an assay sample for detecting influenza virus using immunochromatography, comprising a nonionic surfactant and at least 0.3 M but less than 1.0 M alkali metal ion, wherein the assay sample is prepared by mixing the sample pretreatment solution and a sample selected from the group consisting of nasal discharge, sputum and throat swab.

13. The use of the sample pre-treatment solution of claim 12, wherein the sample pre-treatment solution comprises a thiocyanate compound.

## Patentansprüche

1. Verfahren zum Nachweis des Influenzavirus, das die folgenden Schritte umfasst: Bereitstellen einer Probenvorbehandlungslösung, umfassend ein nicht-ionisches Tensid und wenigstens 0,3 M, aber weniger als 1,0 M, Alkalimetallion; Herstellen einer Assayprobe durch Mischen der genannten Probenvorbehandlungslösung und einer Probe, ausgewählt aus der Gruppe bestehend aus Nasensekret, Sputum oder Rachenabstrich; und Nachweisen des Influenzavirus in der Assayprobe unter Verwendung von Immunchromatographie.

2. Verfahren nach Anspruch 1, wobei das genannte nichtionische Tensid ein Polyoxyethylen-Tensid ist.

3. Verfahren nach Anspruch 2, wobei das genannte Polyoxyethylen-Tensid aus der Gruppe, bestehend aus Polyoxyethylenalkylphenylether, Polyoxyethylenalkylether, Polyoxyethylensorbitanfettsäureester und Polyoxyethylen/Polyoxypropylen-Copolymeren, ausgewählt ist.

4. Verfahren nach einem der Ansprüche 1 bis 3, wobei eine Konzentration der genannten nicht-ionischen Tenside im Bereich von 0,05 bis 2 (V/V)-% liegt.

5. Verfahren nach einem der Ansprüche 1 bis 4, das eine Thiocyanatverbindung enthält.

6. Verfahren nach Anspruch 5, wobei die genannte Thiocyanatverbindung bei einer Konzentration von wenigstens 0,1 M enthalten ist.

7. Verfahren nach Anspruch 5 oder 6, wobei die genannte Thiocyanatverbindung aus der Gruppe, bestehend aus Thiocyansäure, Thiocyanatestern und Thiocyanaten, ausgewählt ist.

8. Verfahren nach einem der Ansprüche 5 bis 7, wobei die genannte Thiocyanatverbindung aus der Gruppe, bestehend aus Natriumthiocyanat, Kaliumthiocyanat, Ammoniumthiocyanat und Guanidinthiocyanat, ausgewählt ist.

9. Verfahren nach einem der Ansprüche 1 bis 8, wobei der genannte Schritt zur Herstellung der Assayprobe Filtern der Mischung über einen Membranfilter mit einem Porendurchmesser von 1 µm einschließt.

10. Influenzavirus-Testkit, umfassend eine Probenvorbehandlungslösung, umfassend ein nicht-ionisches Tensid und wenigstens 0,3 M, aber weniger als 1,0 M, Alkalimetallion, und eine immunchromatographische Vorrichtung zum Nachweis des Influenzavirus.

11. Testkit nach Anspruch 10, wobei die Probenvorbehandlungslösung eine Thiocyanatverbindung enthält.

12. Verwendung einer Probenvorbehandlungslösung zur Herstellung einer Assayprobe zum Nachweis des Influenzavirus unter Verwendung von Immunchromatographie, umfassend ein nicht-ionisches Tensid und wenigstens 0,3 M, aber weniger als 1,0 M, Alkalimetallion, wobei die genannte Assayprobe durch Mischen der Probenvorbehandlungslösung und einer Probe, ausgewählt aus der Gruppe bestehend aus Nasensekret, Sputum und Rachenabstrich, hergestellt wird.

13. Verwendung der Probenvorhandlungslösung nach Anspruch 12, wobei die Probenvorbehandlungslösung eine Thiocyanatverbindung enthält.

## Revendications

1. Procédé pour détecter un virus de grippe, qui comprend les étapes consistant à fournir une solution de prétraitement d'échantillon comprenant un tensioactif non ionique et un ion de métal alcalin au moins 0,3 M mais inférieur à 1,0 M ; à préparer un échantillon pour analyse en mélangeant ladite solution de prétraitement d'échantillon et un échantillon sélectionné dans le groupe consistant en des sécrétions nasales, du crachat et du prélèvement de gorge; et à détecter le virus de grippe dans l'échantillon pour analyse en utilisant de l'immunochromatographie.

2. Procédé selon la revendication 1, dans lequel ledit tensioactif non ionique est un tensioactif à base de polyoxyéthylène.

3. Procédé selon la revendication 2, dans lequel ledit tensioactif à base de polyoxyéthylène est choisi dans le groupe consistant en des alkylphényléthers de polyoxyéthylène, des alkyléthers de polyoxyéthylène, des esters d'acides gras de polyoxyéthylène sorbitane et des copolymères de polyoxyéthylène / polyoxypropylène.

4. Procédé selon l'une quelconque des revendications 1 à 3, dans lequel une concentration dudit tensioactif non ionique se situe entre 0,05 et 2 % (v/v).

5. Procédé selon l'une quelconque des revendications 1 à 4, qui comprend un composé thiocyanate.

6. Procédé selon la revendication 5, dans lequel ledit composé thiocyanate est contenu à une concentration d'au moins 0,1 M.

7. Procédé selon la revendication 5 ou 6, dans lequel ledit composé thiocyanate est sélectionné dans le groupe consistant en de l'acide thiocyanique, des esters de thiocyanate et des thiocyanates.

8. Procédé selon l'une quelconque des revendications 5 à 7, dans lequel ledit composé thiocyanate est sélectionné dans le groupe consistant en du thiocyanate de sodium, du thiocyanate de potassium, du thiocyanate d'ammonium et du thiocyanate de guanidine.

9. Procédé selon l'une quelconque des revendications 1 à 8, dans lequel ladite étape de préparation de l'échantillon pour analyse comprend de la filtration du mélange à travers un filtre à membrane avec un diamètre de pore de 1 µm.

10. Trousse de test de virus de grippe comprenant une solution de prétraitement d'échantillon comprenant un tensioactif non ionique et un ion de métal alcalin au moins 0,3 M mais inférieur à 1,0 M et un dispositif d'immunochromatographie pour détecter un virus de grippe.

11. Trousse de test selon la revendication 10, dans laquelle la solution de prétraitement d'échantillon comprend un composé thiocyanate.

12. Utilisation d'une solution de prétraitement d'échantillon pour préparer un échantillon pour analyse pour détecter un virus de grippe en utilisant de l'immunochromatographie, comprenant un tensioactif non ionique et un ion de métal alcalin au moins 0,3 M mais inférieur à 1,0 M, dans laquelle l'échantillon pour analyse est préparé en mélangeant la solution de prétraitement d'échantillon et un échantillon sélectionné dans le groupe consistant en des sécrétions nasales, du crachat et du prélèvement de gorge.

13. Utilisation de la solution de prétraitement d'échantillon de la revendication 12, dans laquelle la solution de prétraitement d'échantillon comprend un composé thiocyanate.
